# EUROPÄISCHE PATENTSCHRIFT

(11) **EP 1 719 472 B1**
(45) Veröffentlichungstag und Bekanntmachung des Hinweises auf die Patenterteilung: **19.03.2008**
(21) Anmeldenummer: 05009785.6
(22) Anmeldetag: 04.05.2005
(51) Int. Cl.: A61B 19/00

(54) **Vorrichtungen und Verfahren zur automatischen Verifikation, Kalibrierung und Vermessung von Instrumenten für computer-assistierte Chirurgie**
Devices and methods for automatic verification, calibration and measurement of instruments for computer aided surgery
Dispositifs et procédés de vérification, calibration et de mesure d'instruments pour chirurgie assistée par ordinateur

(43) Veröffentlichungstag der Anmeldung: 08.11.2006
(73) Patentinhaber: BrainLAB AG, 85551 Kirchheim/Heimstetten (DE)
(72) Erfinder: Neubauer, Timo, 85622 Feldkirchen (DE); Plassky, Norman, 99099 Erfurt (DE)
(74) Vertreter: Schwabe - Sandmair - Marx

(56) Entgegenhaltungen:
- WO-A-01/67979
- WO-A-97/29710
- US-A1- 2004 039 402
- US-A1- 2004 171 924
- US-A1- 2004 181 144
- US-B1- 6 535 756

## Beschreibung

Die vorliegende Erfindung betrifft Vorrichtungen und Verfahren zur automatischen Verifikation, Kalibrierung und Vermessung von Instrumenten für den Einsatz in computer-assistierter Chirurgie. Dabei können mit Hilfe von optischen Scanverfahren die Geometrie des Instruments und eines durch mindestens zwei Marker gebildetes Referenzsystems, welches permanent oder abnehmbar am Instrument befestigt sein kann, als dreidimensionales Modell ermittelt und anschließend vom Navigationssystem weiterverwendet werden.

Die Genauigkeit und damit Schnelligkeit des Scanprozesses wird maßgeblich über die bereits für dieses Instrument vorhandenen und zugänglichen Informationen gesteuert, wobei mindestens eine Verifikation, durchaus aber eine Kalibrierung und sogar eine vollständige Vermessung der Instrumenteneigenschaften durchgeführt werden kann oder sogar muss.

### Hintergrund

Bei computergestützten Operationen werden dem Operateur die Position und Orientierung von chirurgischen Instrumenten in Beziehung zu den anatomischen Strukturen des Patienten mit Hilfe eines Navigationssystems auf dessen Anzeigevorrichtung dargestellt. Notwendigerweise müssen für eine genaue Darstellung neben den anatomischen Strukturen auch die verwendeten Instrumente durch geeignete Referenzsysteme Verfolgbar sein, was üblicherweise durch aktive oder passive Markerstrukturen erreicht wird, die dem Navigationssystem ihre Position im Operationsfeld durch Emmision oder Reflexion von Infrarotstrahlung offenbaren.

Die anatomischen Strukturen werden zu Beginn durch Verwendung von navigierbaren Zeigegeräten in einem Registrierungsprozess mit den an ihnen befestigten Referenzsystemen in Beziehung gesetzt und sind dadurch im späteren Operationsprozess für das Navigationssystem räumlich verfolgbar.

Gleiches ist für die Instrumente und ihre Refenzsysteme erforderlich, wobei hier die instrumententypischen Funktionselemente von besonderer Bedeutung sind. Dabei handelt es sich zum Beispiel um Werkzeugflächen (Schneiden, Spitzen etc.), die für die Bearbeitung von beispielsweise knöchernen Strukturen benutzt werden, wobei der Chirurg ein Navigationssystem einsetzt, um trotz möglicherweise eingeschränkter Sichtbarkeit genaue Informationen über deren Position und Orientierung zu erhalten. Die Darstellung der räumlichen Lage des Instruments und seiner Funktionselemente auf der Anzeigevorrichtung beruht auf einer Korrelation von hinterlegten Geometriedaten des Instruments und der räumlichen Daten, die vom Navigationssystem über das am Instrument angebrachte Referenzsystem ermittelbar sind. Sobald die hinterlegten Geometriedaten, die hauptsächlich die Funktionselemente in Bezug auf das Referenzsystem beschreiben, von der tatsächlichen Geometrie des Instruments abweichen (z.B. nach Beschädigung des Instruments), führt dies unweigerlich auch zu einer falschen Darstellung der Beziehung zwischen realem Instrument und zu behandelnder anatomischer Struktur, wenn weiterhin die in der Datenbank hinterlegten Informationen über die initiale Gestalt des Instruments verwendet werden.

### Stand der Technik

Gegenwärtig wird die Relation zwischen Funktionselement des Instruments und seinem Referenzsystem einmalig anhand von Fertigungsunterlagen festgelegt, wobei deren Einhaltung durch Vermessung nach dem Fertigungsprozess sichergestellt wird. Die Relation wird im Navigationssystem hinterlegt und für nachfolgende Operationen ausgelesen und verwendet. Diese sogenannten prä-kalibrierten Instrumente (z.B. Bohrführungen) werden mitunter prä-operativ mit navigierbaren Hilfsmitteln verifiziert, um die Maßhaltigkeit zu bestätigen. Bei unzureichender Maßhaltigkeit wird dies dem Benutzer angezeigt, welcher auf den Einsatz des Instruments verzichten sollte, wenn eine nachfolgend beschriebene Kalibrierung nicht möglich ist. Eine Anpassung der Modelldaten an das real existierende, eventuell abweichende Instrument, ist bei der Verifikation nicht möglich. Die erreichbare Genauigkeit der Verifikation hängt zwangsläufig von der Genauigkeit des Navigationssystems ab, da wiederum die jeweiligen Referenzsysteme zueinander in Relation gesetzt werden. Sichtbarkeitsprobleme und mitunter schlechte Handhabbarkeit beim gleichzeitigen Positionieren von Instrument und Hilfsmittel bewirken dabei tendenziell eine Verlängerung der Operationszeit.

Bei einem weiteren Verfahren wird vor dem Gebrauch des Instruments ein Kalibrierungsvorgang des Instruments durchgeführt. Dabei werden die für die Navigation erforderlichen aber noch offenen Werte funktionsrelevanter Parameter des Instruments (Länge, Durchmesser, ...) oder offene Werte für die Zuordung von Referenzsystem zu Funktionselement (z.B. Pfanneneinschläger mit variablen Pfannen) oder die Abweichungen des realen Instruments vom in der Datenbank hinterlegten Modell mittels eines navigierbaren Kalibrierungswerkzeugs bestimmt, vorrübergehend oder permanent im Navigationssystem gespeichert und bei Bedarf ausgelesen. Auch hier wirken sich die vorstehend beschriebenen Sichtbarkeits- und Handhabbarkeitsprobleme negativ auf die Operationszeit aus. Die Kalibrierung funktioniert gut für Instrumente mit einfachen Geometrien der Funktionselemente aber wiederum nur in den Grenzen der vom Navigationssystem erreichbaren Genauigkeit.

Die vollständige Vermessung insbesondere funktionsrelevanter Strukturen eines schwer zu kalibrierenden oder zu verifizierenden Instruments stellt ein drittes Verfahren dar, das gegenwärtig jedoch nicht zum Einsatz kommt, da weder die geeigneten Hilfsmittel zur Verfügung stehen, noch die erforderlichen Prozeduren in einem vertretbaren zeitlichen Rahmen vom OP-Personal durchgeführt werden könnten.

Dokument US-A-2004/0181144 offenbart ein Verfahren und eine Vorrichtung gemäß der Oberbegriffe der Ansprüche 1 und 14.

### Aufgabe der Erfindung

Es ist die Aufgabe der vorliegenden Erfindung, Verfahren und Vorrichtungen zur Verifikation, Kalibrierung und Vermessung von Instrumenten für die computer-assistierte Chirurgie vorzuschlagen, welche eine Erhöhung der Prozesssicherheit und mitunter eine Zeiteinsparung beim Einsatz von navigierten Instrumenten für chirurgische Prozeduren ermöglichen. Dabei soll die Anwendbarkeit nicht auf symmetrische oder anderweitig geometrisch einfache navigierbare Instrumente beschränkt sein, sondern es sollen auch Instrumente mit komplexen Geometrien für den Einsatz in computer-assistierter Chirurgie vorbereitet werden.

Diese Aufgaben werden durch die Gegenstände der unabhängigen Ansprüche gelöst. Vorteilhafte Ausführungsformen ergeben sich aus den Unteransprüchen.

### Allgemeine Beschreibung

Erfindungsgemäß dienen die Verifikation, Kalibrierung und Vermessung dazu, dass dem Navigationssystem möglichst vor Beginn der Operation korrekte Instrumentendaten über die Geometrie von den Funktionselementen und dem Referenzsystem sowie deren Relation zueinander bereitgestellt und im Speicher des Navigationssystems temporär oder permanent hinterlegt werden, wobei auch eine intra-operative Anwendung der Verifikation, Kalibrierung und Vermessung nicht ausgeschlossen werden soll, um zum Beispiel zwischenzeitlich deformierte Instrumente nach der Durchführung der beschriebenen Verfahren erneut benutzen zu können.

Die Instrumentendaten können Angaben über die Lage der Funktionselemente des Instruments (z.B. Instrumentenspitze) in Bezug auf ein fest angebrachtes oder abnehmbares, aus Markern (aktive oder passive Technologie) gebildetes Referenzsystem beinhalten. Die Daten können insbesondere auch angeben, wie die Funktionselemente, wie z.B. die Spitzen des Instruments, geformt sind.

Bei den erfindungsgemäßen Verfahren zur automatischen Verifikation, Kalibrierung oder Vermessung eines navigierbaren Instruments wird mittels einer vorzugsweise berührungslos arbeitenden Scanvorrichtung oder Abtastvorrichtung die Geometrie des Instruments inklusive einem befestigten oder befestigbaren, aus mindestens zwei Markern gebildeten Referenzsystems ermittelt und mit bereits hinterlegten, dem Instrument oder seiner Instrumentengruppe eindeutig zuordenbaren Werten verglichen bzw. bei Vermessung neu abgelegt. Insbesondere können das Referenzsystem oder die Marker dauerhaft an dem Instrument angeordnet sein oder können zeitweise, z.B. während des Scanvorganges, angebracht werden. Die durch das Scannen gewonnenen Oberflächendaten des Instruments werden dabei mittels einer Datenverarbeitungseinheit oder Recheneinheit in ein dreidimensionales Modell des Instruments umgewandelt, anhand dessen die instrumententypischen Kennwerte berechnet werden können. Das dreidimensionale Modell des Instruments kann dabei alle zur Navigation notwendigen Informationen über die Geometrie der Funktionselemente des Instruments und seines Referenzsystems sowie deren Beziehung zueinander beinhalten. Abschließend können die geometrischen Informationen über das Instrument dem Navigationssystem übergeben werden, um dem Operateur die Verwendung des Instruments in der computer-assistierten Chirurgie zu ermöglichen.

### Vorrichtungen zur automatischen Verifikation, Kalibrierung und Vermessung von navigierbaren chirurgischen Instrumenten

Die erfindungsgemäßen Vorrichtungen zur automatischen Verifikation, Kalibrierung und Vermessung von navigierbaren chirurgischen Instrumenten werden nachfolgend beschrieben.

### Scanvorrichtung

Die erfindungsgemäße Scanvorrichtung, die entweder im Gehäuse des Navigationssystems integriert oder in einem separaten Gehäuse untergebracht sein kann, verwendet bekannte 3D-Scantechniken, wie z.B. Lichtschnittverfahren, Objektrasterverfahren, welche eine gerasterte Instrumentenoberfläche erfordern, oder Gitterprojektionsverfahren, bei welchen telezentrisches Gitter auf die Instrumentenoberfläche projiziert werden. Die in der Scanvorrichtung befindliche Scaneinheit arbeitet vorzugsweise optisch, wie mittels eines Lasers, wobei die Oberfläche des Instruments inklusive dauerhaft oder abnehmbar befestigtem Referenzsystem abgetastet wird. Dabei wird das zu scannende Instrument in einem in der Scanvorrichtung enthaltenen Instrumentenhalter befestigt, wobei während das Scanvorgangs dieser Instrumentenhalter relativ zur Scaneinheit bewegt oder gedreht werden kann, um das Instrument der Scaneinheit von allen Seiten zugänglich und somit eine vollständige Digitalisierung der Oberfläche möglich zu machen. Alternativ kann sich auch die Scaneinheit relativ zu einem fixen Instrumentenhalter bewegen oder drehen, wobei in beiden Fällen die Bewegung oder Drehbewegungen über geeignete, in der Scanvorrichtung integrierte Sensorik erfasst oder gesteuert werden kann, um die Winkelstellungen eindeutig mit den Scanergebnissen korrelieren zu können, wodurch ein realitätsgetreues 3D-Model berechenbar wird.

### Markerinspektionseinheit

Zusätzlich kann während das Scanvorgangs mittels einer Infrarot-Kameraeinheit, die separat vorhanden oder in der Scanvorrichtung enthalten sein kann, aber deren Lage in Bezug auf die Scaneinheit fest und bekannt oder ermittelbar ist, ein Emmisions- bzw. Reflexionsmodell der das Referenzsystem bildenden aktiven bzw. passiven Marker bestimmt werden. Die dadurch ermittelten Informationen über die Strahlungseigenschaften der Marker in Abhängigkeit vom Betrachtungswinkel und der sich daraus ergebenden, vom Navigationssystem erfassbaren, optischen Gestalt als räumliche Lage des Referenzsystems, können mit den Ergebnissen aus der Bestimmung seiner geometrischen Gestalt (mittels Scanprozess) korreliert werden. Damit lassen sich während des operativen Einsatzes Betrachtungsfehler des Navigationssystems, die aus sich änderndem Sichtbarkeitsverhalten der Marker in bestimmmten Lagen, z.B. durch partielle Beschädigung der Marker, resultieren können, kompensieren, was eine Erhöhung der Genauigkeit der Darstellung der Lage des Instruments in Bezug auf die zu behandelnde anatomische Struktur auf der Anzeigevorrichtung des Navigationssystems bewirkt. Mit anderen Worten können beim späteren Navigationsvorgang die auf Grund z.B. beschädigter Marker auftretenden Fehlinterpretationen der räumlichen Lage des Referenzsystems durch die Kameraeinheit des Navigationssystems kompensiert und die realitätsgetreue Lage des Instruments insbesondere des Funktionselements oder der Funktionselemente in Bezug auf die anatomische Struktur berechnet und dargestellt werden.

### Datenverarbeitungseinheit

Die erfindungsgemäße Vorrichtung zur automatischen Verifikation, Kalibrierung und Vermessung eines navigierbaren Instruments umfasst weiter eine Recheneinheit oder Datenverarbeitungseinheit, die in die Scanvorrichtung integriert sein kann, aber auch beim Bilden einer separaten Einheit mit dieser drahtlos oder drahtgebunden verbunden sein kann und mit dieser kommunizieren kann. Die von der Scaneinheit erfassten Daten über die Geometrie des navigierbaren Instruments können z.B. an die Datenverarbeitungseinheit gesendet und in dieser weiterverarbeitet werden, indem z.B. die Datenverarbeitungseinheit aus den Ergebnissen des Oberflächenscans ein dreidimensionales Modell des Instruments inklusive des Referenzsystems berechnet. Vorzugsweise kann die Datenverarbeitungseinheit die erfassten Daten oder das erstellte dreidimensionale Modell so auswerten, dass die Geometrie der Funktionselemente des Instruments (z.B. Spitzen) und deren Lage zum Referenzsystem des Instruments ermittelt werden können.

### Datenbank

Weiter kann die erfindungsgemäße Vorrichtung eine Datenbank umfassen, welche mit der Datenverarbeitungseinheit verbunden sein kann, so dass z.B. in die Datenverarbeitungseinheit eingegebene Daten oder an die Datenverarbeitungseinheit gesendete Daten in der Datenbank gespeichert werden können. So können beispielsweise Informationen über die Geometrie der Funktionseinheiten und des Referenzsystems des Instruments sowie deren Lage zueinander für verschiedene Instrumente aus der Datenverarbeitungseinheit in die Datenbank eingespeichert werden, genauso wie Informationen über die Qualität der Marker oder die Eigenschaften des Emmisions- bzw. Reflexionsmodels des instrumentenspezifischen Referenzsystems.

### Anzeigevorrichtung

Auch kann eine Anzeigevorrichtung, insbesondere ein Bildschirm, der mit der Datenverarbeitungseinheit und der Benutzerschnittstelleneinheit drahtgebunden oder drahtlos (WLAN oder Bluetooth) kommuniziert, in der erfindungsgemäßen Vorrichtung vorhanden sein. Die Darstellung der ermittelten Informationen über die Geometrie der Funktionselemente des Instruments und seines Referenzsystems sowie deren Lage zueinander kann in Form von Werten oder grafisch erfolgen. Vorzugsweise wird auf der Anzeigevorrichtung das aus der Datenbank ausgelesene oder durch Verarbeitung der Scannergebnisse ermittelte dreidimensionale Modell des Instruments angezeigt, wobei das Modells zumindest nahezu exakt auf der Anzeigevorrichtung dargestellt wird. Die realitätsnahe Darstellung des dreidimensionalen Modells auf der Anzeigevorrichtung gestattet es dem Benutzer unter Nutzung einer Benutzerschnittstelleneinheit, die vorzugsweise auf der Anzeigevorrichtung angeordnet ist, wie z.B. bei einem Touchscreen, notwendige Befehle für die korrekte Ausführung des erfindungsgemäßen Verfahrens an die Datenverarbeitungseinheit zu übertragen. Diese Befehle können die Auswahl des richtigen Modells aus einer Mehrzahl an ähnlichen Modellvarianten steuern, falls das Instrument nach dem Einbringen in die Scanvorrichtung nicht korrekt oder gar nicht erkannt wird. Weiterhin kann die, nach einem Grobscan mögliche grafische Darstellung von der Scanvorrichtung bis dahin unbekannten Instrumenten dafür genutzt werden, die für das erfolgreiche Durchführen der Instrumentenaufbereitung durch Vermessung erforderlichen Bereiche des Instruments, im besonderen die Funktionselemente und das Referenzsystem, interaktiv zu selektieren. Ebenso lassen sich für neue Instrumente Bezeichnungen eingeben, die das spätere Auffinden in der Datenbank erleichtern.

### Navigationssystemanbindung

Auch kann ein Navigationssystem drahtlos oder drahtgebunden mit der Scanvorrichtung oder der Datenverarbeitungseinheit verbunden sein, so dass die nach den erfindungsgemäßen Verfahren ermittelten Informationen über die Geometrie der Funktionselemente und des Referenzsystems des Instruments sowie deren Lage zueinander, und auch die Qualität der aktiven bzw. passiven Marker und deren daraus resultierendes Emmsions- bzw. Reflexionsmodell an das Navigationssystem, insbesondere an dessen Datenbank übertragen werden können. Mittels der Informationen kann das Navigationssystem den Operateur durch Bereitstellung der zuvor oder gerade ermittelten oder aktuellsten instrumentenspezifischen Daten bei der Durchführung der computer-assistierten Chirurgie unterstützen.

### Verfahren zur automatischen Verifikation, Kalibrierung und Vermessung von navigierbaren chirurgischen Instrumenten

Das erfindungsgemäße Verfahren zur Untersuchung, insbesondere zur automatischen Verifikation, Kalibrierung und Vermessung von navigierbaren chirurgischen Instrumenten mit seinen unterschiedlichen Anforderungen und Bearbeitungsabläufen wird nachfolgend beschrieben.

### Verifikation

Bei der Verifikation sind vorteilhaft die vollständigen geometrischen Daten des Instruments bereits in einer Datenbank des Navigationssystems hinterlegt, was bei sogenannten prä-kalibrierten Instrumenten die Regel ist. Die Durchführung einer Verifikation dieser Instrumente entspricht einer Überprüfung, ob die hinterlegten Daten mit der tatsächlichen Geometrie, insbesondere der Form der Funktionselemente und ihrer Relation zu dem Referenzsystem, übereinstimmen. Ein Unterlassen der Verifikation kann dazu führen, dass die Operation mit einem, nicht unbedingt offensichtlich beschädigten Instrument durchgeführt wird und somit auch die vorhandene Lage des Instruments zur anatomischen Struktur vom Navigationssystem nicht korrekt dargestellt wird.

Das Navigationssystem kann bevorzugt die verwendeten Instrumente anhand der charakteristischen und unterscheidbaren räumlichen Anordnung der das Referenzsystem bildenden Marker identifizieren, was nach einem erfolgreichen Erkennen das Auslesen der instrumentenspezifischen Daten aus der Datenbank zur Weiterverwendung während der Navigation ermöglicht.

Da sich die Scanvorrichtung zur Durchführung der Verifikation im Blickfeld des Kamerasystems befinden kann aber nicht muss und dadurch eine Identifizierung mit Hilfe des Navigationssystems nicht unbedingt sichergestellt werden kann, ist es mitunter sinnvoll auch andere Identifizierungsprozeduren vorzuschlagen, die einen gezielten Abruf der Instrumentendaten durch die Scanvorrichtung ermöglichen. Die Erfüllung der erfindungsgemäßen Aufgabe einer automatischen Verifikation von navigierbaren chirurgischen Instrumenten kann eine Instrumentenidentifizierung mittels Strichcode (Barcode), NFC (Near Field Communication) oder RFID (Radio Frequency Identification) beinhalten, wobei die Informationsträger automatisch beim Einsetzen des Instruments in die Scanvorrichtung erfasst werden können. Diese mobilen Informationsspeicher enthalten entweder selbst die geometrischen Daten des Instruments oder stellen die notwendigen Instrumenteninformationen bereit, anhand derer ein schnelles Auffinden der instrumentenspezifischen Daten in der Datenbank des Navigationssystems möglich ist. Weiterhin ist die Auswahl des zu verfizierenden Instrumentes durch den Benutzer über eine geeignete, insbesondere grafische Schnittstelle denkbar, die entweder mit Hilfe der Anzeigevorrichtung des Navigationssystems oder über eine der Scanvorrichtung zugehörige Anzeigevorrichtung dargestellt werden kann. Ebenso können der Scanvorrichtung die, zum Auffinden der in der Datenbank abgelegten Geometriedaten, erforderlichen Informationen über das Instrument durch eine manualle Eingabe, z.B. mittels eines Instrumentenidentifizierungscodes, übergeben werden. Eine Verifikation ist ebenfalls möglich indem nach Einsetzen des Instruments in die Scanvorrichtung ein Initialscan oder Grobscan mit reduzierter Detailtreue durchgeführt wird, der in kürzester Zeit ein Modell des Instruments mit geringer oder verringerter Auflösung oder eine Grobstruktur des Instruments ermittelt, welches bzw. welche die Suche des Instruments durch Abgleich mit in der Datenbank hinterlegten Instrumentenmodellen gestattet. Auch hier werden nach erfolgreicher Suche die in der Datenbank des Navigationssystem gespeicherten vollständigen Instrumentendaten ausgelesen und bei der anschließenden Verifikation verwendet. Auch kann nach dem Grobscan des Instruments ein Feinscan oder ein weiterer oder zweiter Scanvorgang des Instruments oder Teilen des Instruments mit höherer oder erhöhter Detailtreue durchgeführt werden, woraus ein Modell mit hoher oder höherer Auflösung oder eine detaillierte oder genauere Struktur ermittelt werden kann.

Für alle beschriebenen Varianten der Verifikation ist es vorteilhaft, dass nach der Instrumentenidentifizierung mindestens die Funktionselemente und das Referenzsystem des Instruments, insbesondere deren Form und Lage zueinander, mit hoher Detailtreue gescannt werden, um ausreichend genaue Daten für den Vergleich der in der Datenbank hinterlegten Geometrie mit der tatsächlichen Instrumentengeometrie zu erhalten. Dabei kann erfindungsgemäß eine Erkennungslogik genutzt werden, die selbständig die charakteristischen Instrumenten-elemente identifiziert und damit eine Festlegung des Instrumentenbezugsystems zum Beispiel anhand der das Referenzsystem bildenden Marker festlegen und die Lage darauf bezogener Funktionselemente ermitteln kann. Die Identifizierung des Referenzsystems ist bei der Verwendung von z.B. passiven kugelförmigen Markern durch deren leicht zu erkennende Form, welche sonst eher selten bei chirurgischen Instrumenten vorkommt, sichergestellt. Instrumente mit aktiven Marker, die im Falle einer gewünschten Erfassung des Emmisionsmodells während des Scanvorganges mit einer Energiequelle verbunden sein können, können zum leichteren Auffinden der geometrisch kleinen Dioden (durch die Erkennungslogik nach erfolgtem Grobscan) in deren Umgebung ebenfalls mit geometrisch größeren charakteristischen Formen ausgebildet sein. Bei Nutzen einer externen, durch mit Kabeln verbundenen Energiequelle ist vorzugsweise die Aufnahemvorrichtung ortsfest auszuführen während sich die Scaneinheit relativ zu dieser bewegt.
Bei positivem Ergebnis der Verifikation, was einem Gleichen des Instruments mit dem hinterlegten Modell innerhalb einer vorgegebenen Toleranz entspricht, kann dies dem Navigationssystem über eine geeignete Datenverbindung (drahtgebunden, drahtlos) übermittelt werden und das Instrument kann für die nachfolgende Anwendung freigeschaltet werden.

### Kalibrierung

Die erfindungsgemäße automatische Kalibrierung von navigierbaren chirurgischen Instrumenten ist in seinen Vorraussetzungen und dem Verfahrensablauf identisch mit der vorstehend beschriebenen Verifikation. Während bei der Verifikation nur eine Aussage über die Gleichheit von Modell und realem Instrument getroffen wird, was bei Abweichungen außerhalb oder größer als der zulässigen Toleranz zum Ausschluss des Instruments vom anschließenden Navigationseinsatz bewirkt, werden bei der Kalibrierung die in der Datenbank des Navigationssystems abgespeicherten geometrischen Informationen des Instruments dahingehend korrigiert, dass das Instrument in den außerhalb der zulässigen Toleranzen liegenden Bereichen gescannt wird und die Ergebnisse als aktualisierte Modelldaten in die Datenbank des Navigationssystems übertragen werden.

### Vermessung

Bei der Vermessung ist es nicht erforderlich, dass geometrische Daten des Instruments bereits in einer Datenbank des Navigationssystems hinterlegt sind oder dass das Instrument dem Navigationssystem oder der Scanvorrichtung bekannt ist. Die erfindungsgemäße automatische Vermessung beginnt wie bei dem vorstehend beschriebenen Verfahren damit, dass das zu vermessende, mit passiven oder aktiven Markern ausgerüstete Instrument in dem Instrumentenhalter der Scanvorrichtung befestigt wird. Anschließend kann ein Scannen des Instruments zur Ermittlung eines dreidimensionalen Modells des Instruments oder ein Initialscan oder Grobscan mit reduzierter Detailtreue oder geringer Auflösung durchgeführt werden, wonach mit Hilfe der Datenverarbeitungseinheit ein grobes dreidimensionales Modell des Instruments oder ein Instrumentenmodell mit geringer Auflösung berechnet werden kann, das dann dem Benutzer auf der Anzeigevorrichtung dargestellt werden kann. Durch Nutzung der erfindungsgemäßen Erkennungslogik zum Auffinden charakteristischer, z.B. das Referenzsystem bildenden geometrischen Formen kann die Scanvorrichtung, nach erster Auswertung der Geometriedaten des Initialscans, durch farbliche oder anderweitige Kenntlichmachung dem Benutzer Vorschläge zur Festlegung der das Referenzsystem bildenden Marker und der Funktionselemente des Instruments unterbreiten. Der Benutzer kann die Vorschläge bestätigen oder Verbesserungen mit Hilfe der Benutzerschnittstelleneinheit, die vorzugsweise als Touchscreen ausgeführt ist, durchführen. Die Datenverarbeitungseinheit kann diese Angaben zur Festlegung des Scanbereiches für den nachfolgenden oder zweiten Scanvorgang nutzen, der inbesondere in den definierten Bereichen der Funktionselemente des Instruments und seines Referenzsystems mit hoher oder erhöhter Genauigkeit oder Auflösung durchgeführt werden kann. Die gewonnen Oberflächeninformationen können anschließend drahtgebunden oder drahtlos an die Datenverarbeitungseinheit übertragen werden, welche die exakten geometrischen Werte für die Lage der das Referenzsystem bildenden Marker und deren Abstand zu den Funktionselementen (z.B. Spitzen) des Instruments berechnen kann. Die dadurch bestimmten instrumentenspezifischen Kennwerte können wiederum drahtlos oder drahtgebunden an die Datenbank des Navigationssystems übertragen werden, welches bei Erkennung der charakteristischen, durch mindestens zwei aktive oder passive Marker gebildeten Referenzgeometrie die zugehörigen Informationen über die Lage des Funtkionselements aus der Datenbank auslesen kann. Für eine erfindungsgemäß spätere Verifikation oder Kalibrierung des Instruments ist es denkbar die Instrumentengeometrie in der Datenbank unter einem vom Benutzer über die Benutzerschnittstelleneinheit eingegebenen Namen zu speichern, um das erforderliche Auffinden zu erleichtern. Ebenso ist es denkbar einen bereits angesprochenen mobilen, am Instrument befestigten Datenspeicher der z.B. RFID (Radio Frequency Identification) Technologie verwendet, zu benutzen. Damit können die für spätere, insbesondere auf anderen Scanvorrichtungen durchzuführenden Verifikationen oder Kalibrierungen notwendigen geometrischen Informationen am Instrument gespeichert und automatisch ausgelesen werden.

### Ergebnis der automatischen Verifikation, Kalibrierung und Vermessung von Instrumenten für computer-assistierte Chirurgie

Nach der Durchführung der erfindungsgemäßen automatischen Verifikation, Kalibrierung und/oder Vermessung von navigierbaren chirurgischen Instrumenten kann mit Hilfe des Navigationssystems die Raumposition des mindestens aus zwei aktiven oder passiven Markern gebildeten Referenzsystems des Instruments bestimmt, aus der charakteristischen Lage der Marker zueinander das Instrument identifiziert, aus der Datenbank des Navigationssystems ein vollständiges Modell des Instruments (inklusive Referenzsystem und Funktionselemente) geladen, und/oder seine Lage in Bezug auf die zu behandelnde anatomische Struktur dem Operateur auf der Anzeigevorrichtung des Navigationssystems hochgenau dargestellt werden.

Die Erfindung bezieht sich weiterhin auf ein Computerprogramm, welches, wenn es in einem Computer geladen wird oder auf einem Computer läuft, die wie oben beschriebenen Verfahren durchführt. Des Weiteren bezieht sich die Erfindung auf ein Programmspeichermedium oder ein Computerprogrammprodukt mit einem solchen Programm.

### Bildbeschreibung

Die Erfindung wird nachfolgend anhand bevorzugter Ausführungsbeispiele beschrieben. Es zeigen:
- Figur 1: eine erfindungsgemäße Vorrichtung zur automatischen Verifikation, Kalibrierung und Vermessung eines Instruments 700 mit separater Anzeigevorrichtung 300, sowie separater Datenverarbeitungseinheit 400;
- Figur 2: eine weitere Ausführungsform der erfindungsgemäßen Vorrichtung zur automatischen Verifikation, Kalibrierung und Vermessung eines Instruments 700 mit einer an einem Navigationssystem 600 angebrachten Anzeigevorrichtung 300 und einer in das Navigationssystem 600 integrierten Datenverarbeitungseinheit 400;
- Figur 3: eine erfindungsgemäße Scanvorrichtung mit dem aufzubereitenden Instrument 700; und
- Figur 4: eine erfindungsgemäße Anzeigevorrichtung 300.

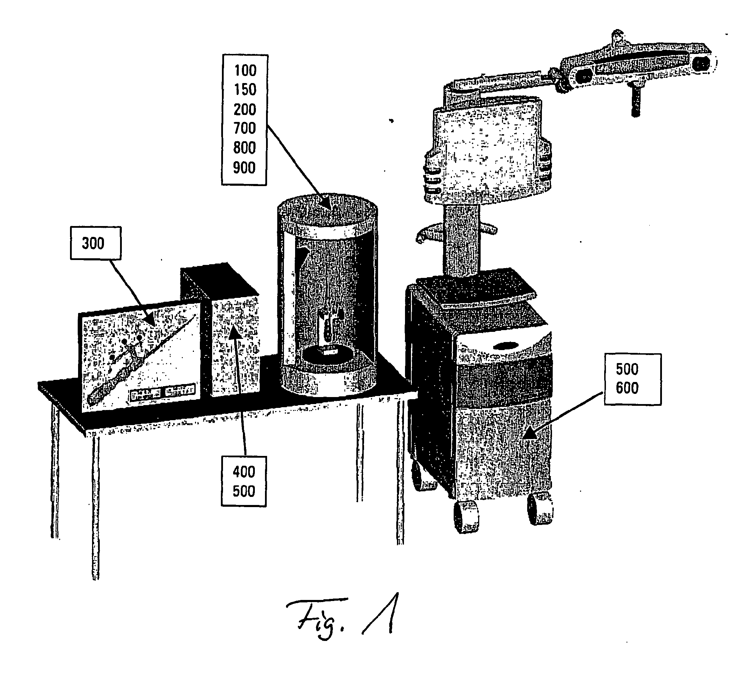

Figur 1 zeigt eine erste Ausführungsform der vorliegenden Erfindung, wobei eine Scanvorrichtung 100 und eine Instrumentenhalteeinheit 200 in einem zylinderförmigen Gehäuse angeordnet sind. Auch kann in dem Gehäuse eine Infrarot-Kameraeinheit 150 angebracht sein, um den Zustand oder die Form oder die Qualität von aktiven oder passiven Markern überprüfen zu können, indem zum Beispiel Infrarot-Strahlung auf die Marker gestrahlt wird und die reflektierte Infrarot-Strahlung von der Infrarot-Kameraeinheit 150 erfasst wird oder indem von den Markern emittierte Infrarot-Strahlung von der Infrarot-Kameraeinheit 150 erfasst wird. Das Instrument 700 ist in der vorliegenden Ausführungsform innerhalb des Gehäuses in der Instrumentenhalteeinheit 200 vorzugsweise fest oder unbeweglich positioniert, wobei auf dem Instrument 700 ein Referenzsystem 800 angebracht ist und das Instrument 700 als Funktionselement eine Instrumentenspitze 900 aufweist.

Die Scanvorrichtung 100 tastet das Instrument vorzugsweise optisch, zum Beispiel mittels eines Lasers oder taktil ab, wobei das Gehäuse der Scanvorrichtung 100 wie in der vorliegenden Ausführungsform offen aber auch geschlossen sein kann. Auch kann die Scanvorrichtung 100 oder der Laser der Scanvorrichtung 100 eine Drehbewegung um das Instrument ausführen oder die Scanvorrichtung 100 oder das Gehäuse der Scanvorrichtung 100 kann so ausgebildet sein, dass eine Drehung der Instrumentenhalteeinheit 200 oder des Instruments 700 ausgeführt werden kann und die Form des Instruments 700 von allen Seiten erfasst wird. Das Gehäuse der Scanvorrichtung 100 besitzt insbesondere eine Größe, auf Grund derer gängige Operationsinstrumente in dem Gehäuse der Scanvorrichtung 100 positioniert und vorzugsweise vollständig abgetastet werden können. Die Infrarot-Kameraeinheit 150, die in der vorliegenden Ausführungsform in oder an dem Gehäuse der Scanvorrichtung 100 angebracht ist, kann die Qualität, insbesondere das gleichmäßige Reflexionsverhalten der Marker untersuchen, indem zum Beispiel die Marker aus verschiedenen Winkeln von der Infrarot-Kameraeinheit 150 bestrahlt werden und aus der Reflexionscharakteristik der Marker ermittelt wird, welchen Zustand oder welche Qualität die Marker aufweisen. So kann zum Beispiel ein gleichmäßiges Reflexionsverhalten der Marker auf einen guten Zustand, oder intakten Zustand oder eine hohe Qualität der Marker hinweisen, während ein ungleichmäßiges Reflexionsverhalten oder eine ungleichmäßige Reflexionscharakteristik, die mit der Infrarot-Kameraeinheit 150 erfasst wurde, auf einen beschädigten Zustand oder eine geringe Qualität der Marker hinweisen kann.

Aus der ermittelten Qualität oder dem Zustand oder der Form der Marker oder des Referenzsystems 800, welche z.B. aus verschiedenen Betrachtungswinkeln mittels der Infrarot-Kameraeinheit erfasst wurden, kann insbesondere die optische Gestalt des Referenzsystems 800 oder der Marker berechnet werden, welche mit den ermittelten Informationen über die geometrische Gestalt des Referenzsystems 800 kombiniert werden kann. Die Korrelation von optischer und geometrischer Gestalt kann genutzt werden, um bei der Navigation, in Abhängigkeit vom Betrachtungswinkel des Referenzsystems durch die Kamera des Navigationsystems auftauchende Fehler aufgrund falscher Berechnung der räumlichen Lage durch z.B. beschädigte Marker zu kompensieren.

Die erfassten Informationen über das Instrument 700 und das Referenzsystem 800, das während des Scanvorganges vorzugsweise an dem Instrument 700 angeordnet ist, und die Position oder Qualität der Marker kann von der Scanvorrichtung 100 an eine Datenverarbeitungseinheit 400 übertragen werden, in welcher die erfassten Scandaten in ein virtuelles Modell umgewandelt werden. Die Datenverarbeitungseinheit 400 kann Funktionselemente oder Funktionseinheiten des Instruments 700 zum Beispiel direkt aus den erfassten Daten über die Geometrie des Instruments 700 und des Referenzsystems 800 erkennen oder ermitteln oder kann zum Beispiel unter Berücksichtigung der Geometrieverhältnisse des Instruments 700, wie der charakteristischen Anordnung der das Referenzsystem 800 bildenden Marker die zugehörigen Funktionselemente ermitteln, indem zum Beispiel die ermittelten Geometrieverhältnisse mit den in einer Datenbank gespeicherten Geometrieverhältnissen verglichen werden. Die Datenbank, in der die Vergleichswerte oder Vergleichsgeometrieverhältnisse gespeichert sein können oder neu ermittelte Geometrieverhältnisse gespeichert werden können, kann insbesondere in der Datenverarbeitungseinheit 400 angeordnet sein. Das von der Datenverarbeitungseinheit 400 errechnete virtuelle dreidimensionale Modell des Instruments 700 oder die ermittelten Informationen über die Geometrie des Instruments 700 und des Referenzsystems 800 können auf einer Anzeigevorrichtung 300, wie einem Touchscreen, angezeigt oder grafisch dargestellt werden. Insbesondere können auch verschiedene Alternativformen oder -geometrien des Instruments 700 oder der Funktionselemente des Instruments 700 angezeigt werden, deren Form oder Geometrie ähnlich den ermittelten Geometrien oder Funktionseinheiten des Instruments sind, woraus ein Benutzer ein Instrument 700 oder ein Funktionselement auswählen kann. Die ermittelten Informationen, wie die Geometrieverhältnisse des Instruments 700 und des Referenzsystems 800 oder das virtuelle Modell des Instruments 700 können von der Datenverarbeitungseinheit 400 drahtgebunden oder drahtlos, wie mittels WLAN oder Bluetooth, über eine Kommunikationseinheit 500, die an der Datenverarbeitungseinheit 400 und dem Navigationssystem 600 angeordnet sein kann, an das Navigationssystem übertragen werden, so dass anhand der ermittelten Informationen über die Geometrie des Instruments 700 ein Navigationsvorgang von dem Navigationssystem 600 durchgeführt werden kann. Bei dem Navigationsvorgang können die ermittelten Informationen über die Geometrie des Instruments 700 und des Referenzsystems 800 berücksichtigt werden und es können auch Informationen über den Zustand oder die Qualität der Marker berücksichtigt werden, so dass zum Beispiel eine Beschädigung der Marker oder Veränderungen der Geometrie oder Beschädigungen des Instruments bei dem Navigationsvorgang berücksichtigt werden können, um ein genauen Navigationsvorgang zu gewährleisten.

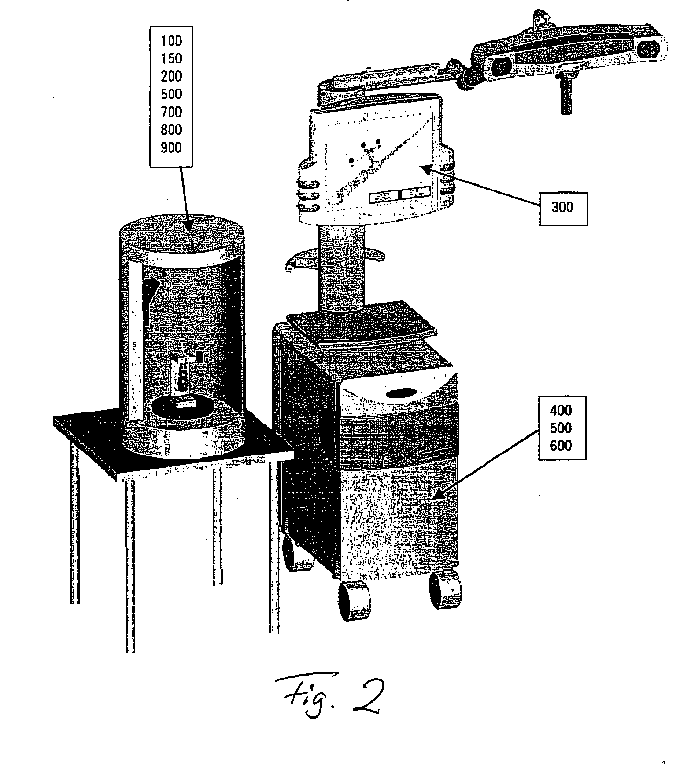

Figur 2 zeigt eine weitere Ausführungsform der vorliegenden Erfindung, wobei die Datenverarbeitungseinheit 400 in dem Navigationssystem 600 integriert ist bzw. in dem Navigationssystem angeordnet ist und die Anzeigevorrichtung 300 in das Navigationssystem 600 integriert ist bzw. an dem Navigationssystem 600 angeordnet ist.

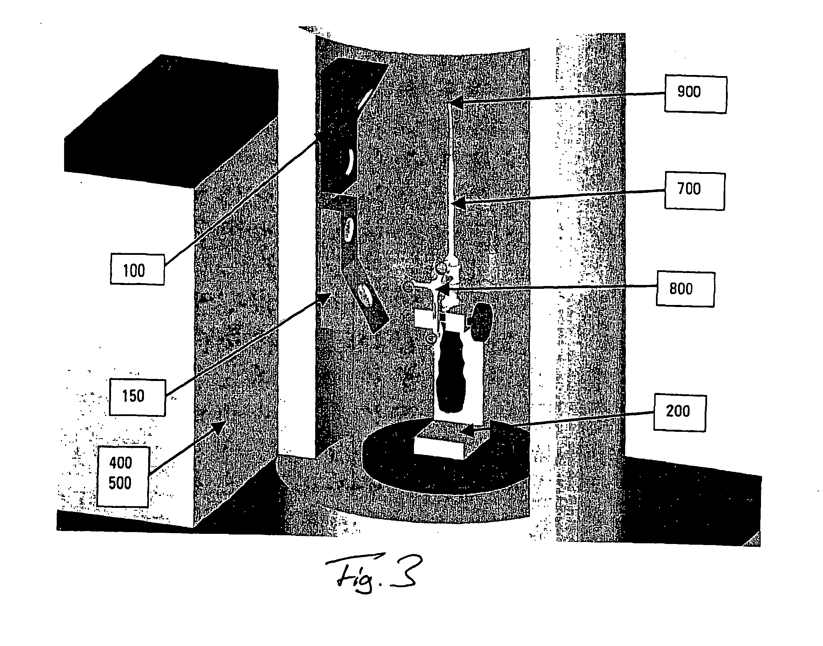

Figur 3 zeigt ein Gehäuse der Scanvorrichtung, in welchem das Instrument 700 positioniert ist, mit einer Scaneinheit 100, einer Infrarot-Kameraeinheit 150 und einem Instrumentenhalter 200, in welchem das Instrument 700 vorzugsweise fest positioniert ist. Das Instrument 700 besitzt als Funktionselement eine Instrumentenspitze 900, deren Form zum Beispiel von der Scaneinheit 100 erfasst werden kann. Auch ist an dem Instrument 700 ein Referenzsystem 800 angeordnet, dessen Form zum Beispiel von der Scaneinheit 100 erfasst werden kann, wobei das Referenzsystem 800 durch Marker gebildet wird, deren Reflexionscharakteristik zum Beispiel von der Infrarot-Kameraeinheit 150 erfasst werden kann.

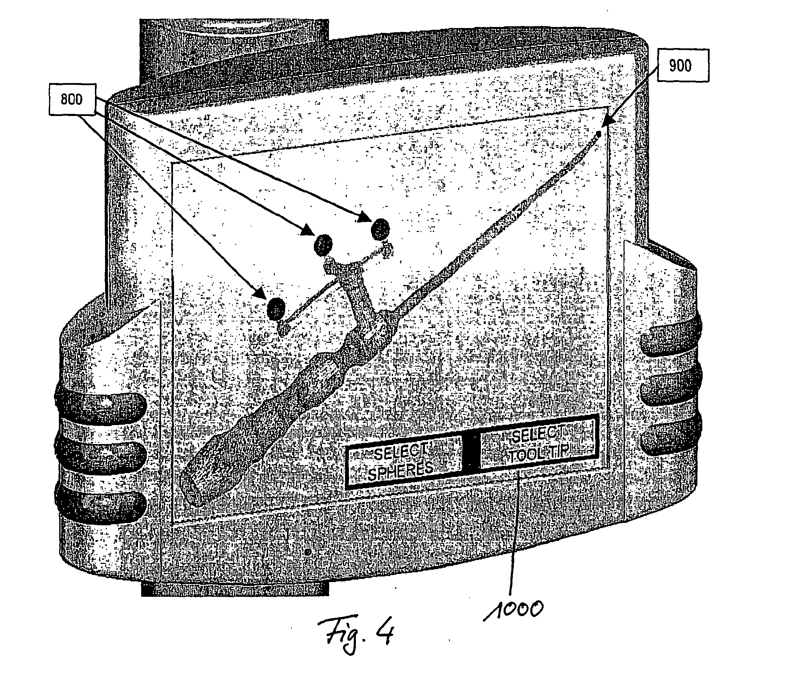

In Figur 4 ist die Anzeigevorrichtung 300 der vorliegenden Erfindung dargestellt, welche das virtuelle ermittelte dreidimensionale Modell des Instruments mitsamt Referenzsystem grafisch darstellt. Mittels einer Benutzerschnittstelleneinheit 1000, die vorzugsweise an der Anzeigevorrichtung 300 angeordnet ist, können zum Beispiel die Marker von einem Benutzer ausgewählt werden oder es können Funktionselemente, wie die Spitze 900 des Instruments, von einem Benutzer ausgewählt werden.

## Patentansprüche

1. Verfahren zur Untersuchung, insbesondere zur automatischen Verifikation, Kalibrierung oder Vermessung eines Instruments (700), wobei mittels einer Scaneinheit (100) die Geometrie des Instruments (700) inklusive eines an dem Instrument (700) anbringbaren, durch mindestens zwei aktive oder passive Marker gebildeten Referenzsystems (800) erfasst werden, und mittels einer Datenverarbeitungseinheit (400) aus den erfassten Informationen über die Geometrie des Instruments (700) und des Referenzsystems (800) ein dreidimensionales Modell des Instruments (700) berechnet wird, wobei anhand der Informationen über die Geometrie des dreidimensionalen Modells des Instruments (700) eine Verifikation, Kalibrierung oder Vermessung des Instruments (700) erfolgt, **dadurch gekennzeichnet,**
**dass** die Emmisions- bzw. Reflexionscharakteristik des aus aktiven bzw. passiven Markern gebildeten Referenzsystems (800) mittels einer Infrarot-Kameraeinheit (150) aus verschiedenen Betrachtungswinkel erfasst wird, aus den Emmisions- bzw. Reflexionseigenschaften von einer Datenverarbeitungseinheit (400) die Qualität oder der Zustand oder die Form des Referenzsystems (800) oder einzelner Marker in Abhängigkeit vom Betrachtungswinkel ermittelt und daraus die optische Gestalt des Referenzsystems berechnet wird, welches mit den ermittelten Informationen über die geometrische Gestalt des Referenzsystems (800) kombiniert wird.

2. Verfahren zur automatischen Verifikation, Kalibrierung oder Vermessung eines Instrumentes (700) nach dem vorhergehenden Anspruch, wobei als Verifikation oder Kalibrierung die Geometrie des Referenzsystems (800) des Instruments (700) oder die Geometrie des Funktionselements des Instruments (700), z.B. die Instrumentenspitze (900), von der Scaneinheit (100) automatisch erkannt werden, die ermittelten Geometrien von einer Datenverarbeitungseinheit (400) mit in einer Datenbank gespeicherten Geometrien von Funktionselementen und Referenzsystemen weiterer Instrumente (700) verglichen werden und aus dem Vergleich automatisch die für das in der Scanvorrichtung vorhandene Instrument (700) hinterlegten Informationen aus der Datenbank abgerufen werden.

3. Verfahren zur automatischen Verifikation, Kalibrierung oder Vermessung eines Instruments (700) nach einem der vorhergehenden Ansprüche, wobei die Oberfläche des Instruments (700) und insbesondere das Referenzsystem (800) sowie das Funktionselement z.B. die Instrumentenspitze (900) von der Scaneinheit (100) erfasst werden und die erfassten Informationen als ein dreidimensionales Modell des Instruments (700) so auf einer Anzeigevorrichtung (300) dargestellt werden, dass das Funktionselement und das Referenzsystem des Instruments (700) von einem Benutzer mittels einer Benutzerschnittstelleneinheit (1000) ausgewählt werden können.

4. Verfahren zur automatischen Verifikation, Kalibrierung oder Vermessung eines Instrumentes (700) nach einem der vorhergehenden Ansprüche, wobei zunächst ein Grobscan des Instruments (700) von der Scaneinheit (100) durchgeführt wird, wobei anhand der mittels des Grobscans erhaltenen Informationen über die Geometrie des Instruments (700) eine Grobstruktur des Instruments (700) ermittelt wird, wobei basierend auf der Grobstruktur des Instruments (700) ein Abgleich mit in der Datenbank hinterlegten Instrumentendaten durchgeführt wird und aus dem Abgleich die für das untersuchte Instrument (700) hinterlegten Informationen aus der Datenbank abgerufen werden.

5. Verfahren zur automatischen Verifikation, Kalibrierung oder Vermessung eines Instrumentes (700) nach einem der vorhergehenden Ansprüche, wobei als Verifikation oder Kalibrierung nach einer Identifizierung des Instruments (700), die Funktionselemente und das Referenzsystem des Instruments (700), insbesondere deren Form und Lage zueinander, mit einem Feinscan gescannt werden, um einen genauen Vergleich zwischen den erfassten Informationen über die Geometrie des Instruments (700) und den in der Datenbank hinterlegten Informationen über die Geometrie des Instruments (700) durchzuführen.

6. Verfahren zur automatischen Verifikation, Kalibrierung oder Vermessung eines Instrumentes (700) nach einem der vorhergehenden Ansprüche, wobei als Verifikation bei Abweichungen zwischen der von der Scaneinheit (100) erfassten Geometrie des Instruments (700) und in der Datenbank hinterlegten Informationen über das Instrument (700) innerhalb einer vorgegebenen Toleranz, ein Übereinstimmen des untersuchten Instruments (700) und des in der Datenbank hinterlegten Instruments (700) angenommen wird und dem Navigationssystem (600) ein positives Ergebnis der Verifikation übermittelt wird, worauf das Instrument (700) für die nachfolgende Anwendung freigeschaltet wird.

7. Verfahren zur automatischen Verifikation, Kalibrierung oder Vermessung eines Instrumentes (700) nach einem der vorhergehenden Ansprüche, wobei als Kalibrierung bei Abweichungen zwischen der von der Scaneinheit (100) erfassten Geometrie des Instruments (700) und in der Datenbank hinterlegten Informationen über das Instrument (700) größer als eine vorgegebene Toleranz, die in der Datenbank des Navigationssystems (600) abgespeicherten Informationen über die Geometrie des Instruments (700) mit den ermittelten Informationen über die Geometrie des Instruments (700) korrigiert werden oder das Instrument (700) in den außerhalb der Toleranz liegenden Bereichen des Instruments (700) erneut gescannt wird und die mittels des zweiten Scanvorgangs ermittelten Informationen in die Datenbank des Navigationssystems (600) übertragen werden.

8. Verfahren zur automatischen Verifikation, Kalibrierung oder Vermessung eines Instrumentes (700) nach einem der vorhergehenden Ansprüche, wobei das Instrument (700) von der Scaneinheit (100) erfasst wird, von der Datenverarbeitungseinheit (400) der Abstand insbesondere zwischen dem Funktionselement (900) und dem Referenzsystem (800) berechnet und an ein Navigationssystem (600) gesendet oder in dessen Datenbank gespeichert wird.

9. Verfahren zur automatischen Verifikation, Kalibrierung oder Vermessung eines Instrumentes (700) nach einem der vorhergehenden Ansprüche, wobei als Vermessung zunächst ein Grobscan des Instruments (700) und Referenzsystems (800) durchgeführt wird, wobei mit den erfassten Informationen ein grobes dreidimensionales Modell des Instruments (700) berechnet wird, das auf einer Anzeigevorrichtung (300) dargestellt wird, worauf eine Erkennungslogik charakteristische Formen, insbesondere das Referenzsystem (800) oder Funktionselemente bildende geometrische Formen, auffinden kann und mittels der gefundenen Formen ein Scanbereich ermittelt wird, innerhalb dessen ein erneuter Scanvorgang mit hoher Genauigkeit durchgeführt wird.

10. Verfahren zur automatischen Verifikation, Kalibrierung oder Vermessung eines Instruments (700) nach einem der vorhergehenden Ansprüche, wobei eine relative Drehbewegung des Instruments (700) bezüglich der Scaneinheit (100) und eine Erfassung der Oberfläche des Instruments (700) während der relativen Drehbewegung durchgeführt wird, wobei aus den Informationen über die Drehbewegung und den ermittelten Informationen über die Oberfläche des Instruments (700) das dreidimensionale Modell des Instruments (700) mit Hilfe der Datenverarbeitungseinheit (400) berechenbar wird.

11. Verfahren zur Navigation eines Instruments (700), wobei nach einer Verifikation, Kalibrierung oder Vermessung des Instruments (700) nach einem der vorhergehenden Ansprüche, wobei das Navigationssystem (600) die Raumposition des Referenzsystems (800) erfasst und aus den in der Datenbank hinterlegten Geometrieverhältnissen des Referenzsystems (800) auch die räumliche Lage des Funktionselements (900) des Instruments (700) bestimmen und dadurch auch dessen räumliche Beziehung zu der zu behandelnden anatomischen Struktur berechnen und dem Benutzer ausgeben kann.

12. Computerprogramm, welches, wenn es auf einem Computer läuft oder in einen Computer geladen ist, die Verfahren nach einem der vorhergehenden Ansprüche durchführt.

13. Programmspeichermedium oder Computerprogrammprodukt mit dem Computerprogramm nach dem vorhergehenden Anspruch.

14. Vorrichtung zur automatischen Verifikation, Kalibrierung oder Vermessung eines Instrumentes (700) mit:
- einer Scaneinheit (100), welche die Geometrie des Instruments (700) und eines an dem Instrument (700) anbringbaren Referenzsystems (800) relativ zu der Geometrie des Instruments (700) erfassen kann, wobei insbesondere eine relative Drehbewegung des Instruments (700) bezüglich der Scaneinheit (100) durchgeführt werden kann; und
- einer Datenverarbeitungseinheit (400), welche aus den erfassten Informationen über die Geometrie des Instruments (700) insbesondere der Anordnung der das Referenzsystem (800) bildenden Marker ein dreidimensionales Modell berechnen kann und über die charakteristische Anordnung der Marker des Referenzsystems insbesondere automatisch die Lage des Funktionselementes des Instruments (700) ermitteln kann, **dadurch gekennzeichnet, dass** die Vorrichtung weiter umfasst:
- eine Infrarot-Kameraeinheit (150) deren räumliche Lage zur Scaneinheit (100) bekannt oder bestimmbar ist und die es ermöglicht eine Emissions- oder Reflexionscharakteristik der Marker und des daraus gebildeten Referenzsystems zu bestimmen, woraus Informationen über den Zustand oder die Qualität oder die Form der Marker aus verschiedenen Blickrichtungen auf das Referenzsystem (800) ermittelt und diese mit den Informationen über die Geometrie des Referenzsystems kombiniert und in einer Datenbank abgelegt werden können.

15. Vorrichtung zur automatischen Verifikation, Kalibrierung oder Vermessung eines Instruments (700) nach dem vorhergehenden Anspruch, mit einer Datenbank, wobei die ermittelten Informationen über die Geometrie des Instruments (700) inklusive des Referenzsystems (800), insbesondere dessen Abstand zu dem Funktionselement (900), wie z.B. der Spitze des Instruments, oder über den Zustand oder die Qualität oder die Form der Marker in der Datenbank gespeichert werden können oder Informationen über die Geometrie weiterer Instrumente inklusive deren Referenzsysteme, insbesondere deren Abstand zu dem Funktionselement, wie z.B. der Spitze weiterer Instrumente oder über den Zustand oder die Qualität oder die Form der Marker weiterer Instrumente in der Datenbank gespeichert sein können.

16. Vorrichtung zur automatischen Verifikation, Kalibrierung oder Vermessung eines Instrumentes (700) nach einem der zwei vorhergehenden Ansprüche mit:
- einer Anzeigevorrichtung (300), insbesondere einem Touchscreen, auf welcher das ermittelte dreidimensionale Modell des Instruments (700) dargestellt werden kann; und
- einer Benutzerschnittstelleneinheit (1000), welche insbesondere an der Anzeigevorrichtung (300), z.B. als Touchscreen, angeordnet ist, mittels welcher das Funktionselement (900) des Instruments (700), z.B. die Instrumentenspitze (900) oder die, das Referenzsystem (800) bildenden Marker, ausgewählt werden können.

17. Vorrichtung zur automatischen Verifikation, Kalibrierung oder Vermessung eines Instrumentes (700) nach einem der drei vorhergehenden Ansprüche mit einem Navigationssystem (600), welches mit der Datenverarbeitungseinheit (400) der Scanvorrichtung drahtlos oder drahtgebunden verbunden ist, wobei an das Navigationssystem (600) die ermittelten Informationen über das Instrument, insbesondere die Geometrie des Funktionselements (900) und des Referenzsystems (800) sowie deren relative Lage zueinander, gesendet werden, wobei mit dem Navigationssystem (600) anhand der übermittelten Informationen über die Anordnung der das Referenzsystem bildenden Marker, unter Einbeziehung und gegebenenfalls Kompensation der ermittelten Fehler der Marker, ein Navigationsvorgang durchgeführt werden kann.

18. Vorrichtung zur automatischen Verifikation, Kalibrierung oder Vermessung eines Instrumentes (700), das zur Durchführung des Verfahrens nach einem der Ansprüche 1 bis 11 mit Instrumenten verwendet wird, die mit einem mobilen, nur lesbaren oder lesbaren und beschreibbaren, insbesondere drahtlosen Übertragungstechnologien, wie RFID, verwendenden Datenspeicher ausgerüstet sind, um die für die erfindungsgemäßen Verfahren erforderlichen Identifizierungsmerkmale bereitzustellen und gegebenenfalls die Ergebnisse nach Durchführung der erfindungsgemäßen Verfahren am Instrument speichern zu können.

19. Computerprogramm nach Anspruch 12, das charakteristische geometrische Strukturen des Instruments (700), insbesondere die Form der Funktionselemente (900) und des Referenzsystems (800) oder die das Referenzsystem bildenden Marker mit Hilfe eines erfindungsgemäßen Erkennungsalgorithmus automatisch bestimmt, wodurch eine Identifizierung möglich ist und die zur Durchführung der automatischen Verifikation, Kalibrierung oder Vermessung des Instruments notwendigen Informationen aus der Datenbank abgerufen werden können, wobei ebenfalls die automatische Erkennung der charakteristischen geometrischen Strukturen dazu genutzt werden kann, um dem Benutzer mit Hilfe der Anzeigevorrichtung Vorschläge für die, mit hoher Detailtreue zu scannende Bereiche zu unterbreiten.

## Claims

1. A method for examining, in particular for automatically verifying, calibrating or surveying an instrument (700), wherein by means of a scanning unit (100), the geometry of the instrument (700) - including a reference system (800) which is mountable on the instrument (700) and formed by at least two active or passive markers - is detected, and by means of a data processing unit (400), a three-dimensional model of the instrument (700) is calculated from the detected information concerning the geometry of the instrument (700) and of the reference system (800), wherein on the basis of the information concerning the geometry of the three-dimensional model of the instrument (700), the instrument (700) is verified, calibrated or surveyed, **characterised in that**:
the emission characteristics and/or reflection characteristics of the reference system (800) formed from active and/or passive markers is recorded from different angles of observation by means of an infrared camera unit (150); from the emission properties and/or reflection properties, the quality or the condition or the shape of the reference system (800) or of individual markers are ascertained by a data processing unit (400) depending on the angle of observation; and from this, the optical outline of the reference system is calculated and combined with the ascertained information concerning the geometric outline of the reference system (800).

2. The method for automatically verifying, calibrating or surveying an instrument (700) according to the preceding claim, wherein: as a verification or calibration, the geometry of the reference system (800) of the instrument (700) or the geometry of the functional element of the instrument (700), e.g. of the instrument tip (900), is automatically recognised by the scanning unit (100); a data processing unit (400) compares the ascertained geometries with geometries of functional elements and reference systems of other instruments (700), saved in a database; and from the comparison, the information stored for the instrument (700) provided in the scanning device is automatically retrieved from the database.

3. The method for automatically verifying, calibrating or surveying an instrument (700) according to any one of the preceding claims, wherein the surface of the instrument (700) and in particular the reference system (800) and the functional element, e.g. the instrument tip (900), are detected by the scanning unit (100), and the detected information is represented on a display device (300) as a three-dimensional model of the instrument (700), such that the functional element and the reference system of the instrument (700) can be selected by a user by means of a user interface unit (1000).

4. The method for automatically verifying, calibrating or surveying an instrument (700) according to any one of the preceding claims, wherein a rough scan of the instrument (700) is initially carried out by the scanning unit (100), and on the basis of the information, obtained by means of the rough scan, concerning the geometry of the instrument (700), a rough structure of the instrument (700) is ascertained, wherein based on the rough structure of the instrument (700), a comparison is made with instrument data stored in the database, and from the comparison, the information stored for the instrument (700) being examined is retrieved from the database.

5. The method for automatically verifying, calibrating or surveying an instrument (700) according to any one of the preceding claims, wherein as a verification or calibration, and after identifying the instrument (700), the functional elements and the reference system of the instrument (700), in particular their shape and position with respect to one another, are scanned using a fine scan, in order to make a precise comparison between the detected information concerning the geometry of the instrument (700) and the information, stored in the database, concerning the geometry of the instrument (700).

6. The method for automatically verifying, calibrating or surveying an instrument (700) according to any one of the preceding claims, wherein as a verification and if the geometry of the instrument (700) detected by the scanning unit (100) and information concerning the instrument (700) stored in the database deviate within a given tolerance level; the instrument (700) being examined and the instrument (700) stored in the database are assumed to match and a positive verification result is transmitted to the navigation system (600), whereupon the instrument (700) is activated for the subsequent application.

7. The method for automatically verifying, calibrating or surveying an instrument (700) according to any one of the preceding claims, wherein as a calibration and if the geometry of the instrument (700) detected by the scanning unit (100) and information concerning the instrument (700) stored in the database deviate to a larger extent than a given tolerance level, the information, saved in the database of the navigation system (600), concerning the geometry of the instrument (700) is corrected using the ascertained information concerning the geometry of the instrument (700), or the instrument (700) is newly scanned in the areas of the instrument (700) lying beyond the tolerance level and the information ascertained by means of the second scanning procedure is transferred into the database of the navigation system (600).

8. The method for automatically verifying, calibrating or surveying an instrument (700) according to any one of the preceding claims, wherein the instrument (700) is detected by the scanning unit (100) and in particular the distance between the functional element (900) and the reference system (800) is calculated by the data processing unit (400) and transmitted to a navigation system (600) or saved in its database.

9. The method for automatically verifying, calibrating or surveying an instrument (700) according to any one of the preceding claims, wherein as a survey, a rough scan of the instrument (700) and of the reference system (800) is initially carried out, wherein using the information detected, a rough three-dimensional model of the instrument (700) is calculated which is represented on a display device (300), on which a recognition logic can locate characteristic shapes, in particular geometric shapes forming the reference system (800) or functional elements, and by means of the shapes found, a scanning area is ascertained within which a new, high-precision scanning procedure is carried out.

10. The method for automatically verifying, calibrating or surveying an instrument (700) according to any one of the preceding claims, wherein the instrument (700) is rotated-relative to the scanning unit (100) and the surface of the instrument (700) is detected during the relative rotation, wherein the three-dimensional model of the instrument (700) can be calculated with the aid of the data processing unit (400) from the information concerning the rotation and the ascertained information concerning the surface of the instrument (700).

11. A method for navigating an instrument (700), wherein once the instrument (700) has been verified, calibrated or surveyed according to any one of the preceding claims, the navigation system (600) detects the spatial position of the reference system (800) and, from the geometrical relations of the reference system (800) stored in the database, can also determine the spatial position of the functional element (900) of the instrument (700) and can thus also calculate and output to the user its spatial relation to the anatomical structure to be treated.

12. A computer program which, when running on a computer or loaded onto a computer, carries out the methods according to any one of the preceding claims.

13. A program storage medium or computer program product comprising the computer program according to the preceding claim.

14. A device for automatically verifying, calibrating or surveying an instrument (700), comprising:
- a scanning unit (100) which can detect the geometry of the instrument (700) and of a reference system (800) attachable on the instrument (700), relative to the geometry of the instrument (700), wherein in particular the instrument (700) can be rotated relative to the scanning unit (100); and
- a data processing unit (400) which, from the detected information concerning the geometry of the instrument (700), in particular the arrangement of the markers forming the reference system (800), can calculate a three-dimensional model and can ascertain the position of the functional element of the instrument (700), in particular automatically, via the characteristic arrangement of the markers of the reference system;
**characterised in that** the device further comprises:
- an infrared camera unit (150), the spatial position of which with respect to the scanning unit (100) is known or can be determined and which enables emission characteristics or reflection characteristics of the markers and of the reference system formed by the markers to be determined, from which information concerning the condition or the quality or the shape of the markers can be ascertained from various directions of view onto the reference system (800) and combined with the information concerning the geometry of the reference system and stored in a database.

15. The device for automatically verifying, calibrating or surveying an instrument (700) according to the preceding claim, comprising a database, wherein the ascertained information concerning the geometry of the instrument (700) including the reference system (800), in particular its distance from the functional element (900) such as for example the tip of the instrument, or concerning the condition or the quality or the shape of the markers can be saved in the database, or information concerning the geometry of other instruments, including their reference systems, in particular their distance from the functional element such as for example the tip of other instruments, or concerning the condition or the quality or the shape of the markers of other instruments can be saved in the database.

16. The device for automatically verifying, calibrating or surveying an instrument (700) according to any one of the preceding two claims, comprising:
- a display device (300), in particular a touch screen, on which the ascertained three-dimensional model of the instrument (700) can be represented; and
- a user interface unit (1000) which is arranged in particular on the display device (300), e.g. as a touch screen, by means of which the functional element (900) of the instrument (700), e.g. the instrument tip (900) or the markers forming the reference system (800), can be chosen.

17. The device for automatically verifying, calibrating or surveying an instrument (700) according to any one of the preceding three claims, comprising a navigation system (600) linked wirelessly or via a wire connection to the data processing unit (400) of the scanning device, wherein the ascertained information concerning the instrument, in particular the geometry of the functional element (900) and of the reference system (800) and their position with respect to each other, can be transmitted to the navigation system (600), and on the basis of the transferred information concerning the arrangement of the markers forming the reference system, a navigation procedure can be carried out using the navigation system (600) and incorporating and where appropriate compensating for the ascertained errors of the markers.

18. A device for automatically verifying, calibrating or surveying an instrument (700), used for carrying out the method according to any one of claims 1 to 11, using instruments equipped with a mobile, read-only or readable and writeable data memory which in particular uses wireless transfer technologies such as RFID, in order to provide the identifying features required for the methods in accordance with the invention and, where appropriate, in order to be able to save the results on the instrument, after carrying out the methods in accordance with the invention.

19. The computer program according to claim 12, which automatically determines characteristic geometric structures of the instrument (700), in particular the shape of the functional elements (900) and of the reference system (800) or the markers forming the reference system, with the aid of a recognition algorithm in accordance with the invention, whereby an identification is possible and the information necessary for automatically verifying, calibrating or surveying the instrument can be retrieved from the database, wherein automatically recognising the characteristic geometric structures can also be used to make suggestions, with the aid of the display device, to the user for the areas to be scanned at a high level of detail.

## Revendications

1. Procédé d'examen, en particulier pour la vérification, l'étalonnage ou la mesure automatique d'un instrument (700), par lequel au moyen d'une unité d'analyse (100) on détecte la géométrie de l'instrument (700) y compris d'un système de référence (800) qui peut être placé sur l'instrument (700) et qui est formé par au moins deux repères actifs ou passifs, et au moyen d'une unité de traitement de données (400) on calcule, à partir des informations relevées relatives à la géométrie de l'instrument (700) et du système de référence (800), un modèle tridimensionnel de l'instrument (700), une vérification, étalonnage ou mesure de l'instrument (700) étant effectué à l'aide des informations relatives à la géométrie du modèle tridimensionnel de l'instrument (700),
**caractérisé**
**en ce que** la caractéristique d'émission ou de réflexion du système de référence (800), formé par des repères actifs ou passifs, est relevée au moyen d'une unité de caméra à infrarouge (150) à partir de différents angles d'observation, à partir des propriétés d'émission ou de réflexion, on détermine, par une unité de traitement de données (400), la qualité ou l'état ou la forme du système de référence (800) ou de repères individuels, en fonction de l'angle d'observation, et à partir de ceci on calcule la forme optique du système de référence qui est combinée aux informations déterminées relatives à la forme géométrique du système de référence (800).

2. Procédé pour la vérification, l'étalonnage ou la mesure automatique d'un instrument (700) selon la revendication précédente, dans lequel, comme vérification ou étalonnage, l'unité d'analyse (100) reconnaît automatiquement la géométrie du système de référence (800) de l'instrument (700) ou la géométrie de l'élément fonctionnel de l'instrument (700), par exemple la pointe (900) de l'instrument, une unité de traitement de données (400) compare les géométries déterminées à des géométries, mémorisées dans une banque de données, d'éléments fonctionnels et de systèmes de référence d'autres instruments (700), et à partir de la comparaison on appelle automatiquement de la banque de données les informations enregistrées pour l'instrument (700) se trouvant dans le dispositif d'analyse.

3. Procédé pour la vérification, l'étalonnage ou la mesure automatique d'un instrument (700) selon l'une des revendications précédentes, dans lequel l'unité d'analyse (100) détecte la surface de l'instrument (700) et en particulier le système de référence (800) ainsi que l'élément fonctionnel, par exemple la pointe (900) de l'instrument, et on représente les informations relevées sous la forme d'un modèle tridimensionnel de l'instrument (700), sur un dispositif d'affichage (300), de manière que l'élément fonctionnel et le système de référence de l'instrument (700) puissent être sélectionnés par un utilisateur au moyen d'une unité d'interface d'utilisateur (1000).

4. Procédé pour la vérification, l'étalonnage ou la mesure automatique d'un instrument (700) selon l'une des revendications précédentes, dans lequel l'unité d'analyse (100) procède d'abord une analyse grossière de l'instrument (700), on détermine, à l'aide des informations relatives à la géométrie de l'instrument (700), obtenues au moyen de l'analyse grossière, une structure grossière de l'instrument (700), et sur la base de la structure grossière de l'instrument (700) on procède à un ajustement avec des données d'instrument enregistrées dans la banque de données, et à partir de l'ajustement on appelle de la banque de données les informations enregistrées pour l'instrument (700) analysé.

5. Procédé pour la vérification, l'étalonnage ou la mesure automatique d'un instrument (700) selon l'une des revendications précédentes, dans lequel comme vérification ou étalonnage, après identification de l'instrument (700), on analyse avec une analyse fine les éléments fonctionnels et le système de référence de l'instrument (700), en particulier leur forme et leur position relative, pour procéder à une comparaison précise entre les informations relevées relatives à la géométrie de l'instrument (700) et les informations enregistrées dans la banque de données relatives à la géométrie de l'instrument (700).

6. Procédé pour la vérification, l'étalonnage ou la mesure automatique d'un instrument (700) selon l'une des revendications précédentes, dans lequel, comme vérification, en cas d'écarts entre la géométrie de l'instrument (700) relevée par l'unité d'analyse (100) et des informations relatives à l'instrument (700), enregistrées dans la banque de données, à l'intérieur d'une tolérance prédéfinie, on admet une coïncidence de l'instrument examiné (700) et de l'instrument (700) enregistré dans la banque de données, et on transmet au système de navigation (600) un résultat positif de la vérification, après quoi l'instrument (700) est autorisé pour l'application suivante.

7. Procédé pour la vérification, l'étalonnage ou la mesure automatique d'un instrument (700) selon l'une des revendications précédentes, dans lequel, comme étalonnage, en cas d'écarts entre la géométrie de l'instrument (700) relevée par l'unité d'analyse (100) et des informations relatives à l'instrument (700), enregistrées dans la banque de données, supérieurs à une tolérance prédéfinie, on corrige les informations mémorisées dans la banque de données du système de navigation (600), relatives à la géométrie de l'instrument (700), avec les informations déterminées relatives à la géométrie de l'instrument (700), ou on analyse à nouveau l'instrument (700) dans les zones de l'instrument (700) situées à l'extérieur de la tolérance, et on transmet les informations déterminées au moyen de la seconde analyse dans la banque de données du système de navigation (600).

8. Procédé pour la vérification, l'étalonnage ou la mesure automatique d'un instrument (700) selon l'une des revendications précédentes, dans lequel l'unité d'analyse (100) détecte l'instrument (700), l'unité de traitement de données (400) calcule la distance en particulier entre l'élément fonctionnel (900) et le système de référence (800), laquelle est envoyée à un système de navigation (600) ou mémorisée dans sa banque de données.

9. Procédé pour la vérification, l'étalonnage ou la mesure automatique d'un instrument (700) selon l'une des revendications précédentes, dans lequel, pour la mesure on procède d'abord à une analyse grossière de l'instrument (700) et du système de référence (800), et avec les informations relevées on calcule un modèle tridimensionnel grossier de l'instrument (700) qui est représenté sur un dispositif d'affichage (300), après quoi une logique de reconnaissance peut trouver des formes caractéristiques, en particulier des formes géométriques formant le système de référence (800) ou des éléments fonctionnels, et au moyen des formes trouvées on détermine une zone d'analyse à l'intérieur de laquelle on procède avec une grande précision à une nouvelle analyse.

10. Procédé pour la vérification, l'étalonnage ou la mesure automatique d'un instrument (700) selon l'une des revendications précédentes, dans lequel on procède à un mouvement de rotation relatif de l'instrument (700) par rapport à l'unité d'analyse (100) et à une détection de la surface de l'instrument (700) pendant le mouvement de rotation relatif, et à partir des informations relatives au mouvement de rotation et des informations déterminées relatives à la surface de l'instrument (700), on peut calculer le modèle tridimensionnel de l'instrument (700) à l'aide de l'unité de traitement de données (400).

11. Procédé pour la navigation d'un instrument (700), par lequel après une vérification, étalonnage ou mesure de l'instrument (700) selon l'une des revendications précédentes, dans lequel le système de navigation (600) détecte la position spatiale du système de référence (800) et peut déterminer, à partir des relations géométriques, enregistrées dans la banque de données, du système de référence (600), également la position spatiale de l'élément fonctionnel (900) de l'instrument (700), et peut calculer à partir de là aussi sa relation spatiale à la structure anatomique à traiter, et peut la fournir à l'utilisateur.

12. Programme d'ordinateur qui, lorsqu'il tourne sur un ordinateur ou est chargé dans un ordinateur, met en oeuvre les procédés selon l'une des revendications précédentes.

13. Moyen de mémorisation de programme ou produit de programme d'ordinateur avec le programme d'ordinateur selon la revendication précédente.

14. Dispositif pour la vérification, l'étalonnage ou la mesure automatique d'un instrument (700) comportant :
- une unité d'analyse (100) qui peut détecter la géométrie de l'instrument (700) et d'un système de référence (800) à placer sur l'instrument (700), par rapport à la géométrie de l'instrument (700), un mouvement de rotation relatif de l'instrument (700) par rapport à l'unité d'analyse (100) pouvant en particulier être exécuté ; et
- une unité de traitement de données (400) qui, à partir des informations relevées relatives à la géométrie de l'instrument (700), en particulier de l'agencement des repères formant le système de référence (800), peut calculer un modèle tridimensionnel et peut déterminer, à travers l'agencement caractéristique des repères du système de référence, en particulier automatiquement, la position de l'élément fonctionnel de l'instrument (700),
**caractérisé en ce que** le dispositif comprend en outre :
- une unité de caméra à infrarouge (150) dont la position spatiale par rapport à l'unité d'analyse (100) est connue ou peut être déterminée et qui permet de déterminer une caractéristique d'émission ou de réflexion des repères et du système de référence formé à partir de ceux-ci, à partir de laquelle on détermine des informations relatives à l'état ou à la qualité ou à la forme des repères à partir de différentes directions d'observation sur le système de référence (800), et on peut combiner celles-ci aux informations relatives à la géométrie du système de référence et les enregistrer dans une banque de données.

15. Dispositif pour la vérification, l'étalonnage ou la mesure automatique d'un instrument (700) selon la revendication précédente, comportant une banque de données, les informations déterminées relatives à la géométrie de l'instrument (700), y compris du système de référence (800), en particulier sa distance à l'élément fonctionnel (900), tel que par exemple la pointe de l'instrument, ou relatives à l'état ou à la qualité ou à la forme des repères pouvant être mémorisés dans la banque de données, ou des informations relatives à la géométrie d'autres instruments, y compris de leurs systèmes de référence, en particulier leur distance par rapport à l'élément fonctionnel, tel que par exemple la pointe d'autres instruments, ou relatives à l'état ou à la qualité ou à la forme des repères d'autres instruments, peuvent être mémorisées dans la banque de données.

16. Dispositif pour la vérification, l'étalonnage ou la mesure automatique d'un instrument (700) selon l'une des deux revendications précédentes, comportant :
- un dispositif d'affichage (300), en particulier un écran tactile, sur lequel le modèle tridimensionnel déterminé de l'instrument (700) peut être représenté ; et
- une unité d'interface utilisateur (1000) qui est disposée en particulier sur le dispositif d'affichage (300), par exemple sous la forme d'un écran tactile, au moyen de laquelle on peut sélectionner l'élément fonctionnel (900) de l'instrument (700), par exemple la pointe (900) de l'instrument ou les repères formant le système de référence (800).

17. Dispositif pour la vérification, l'étalonnage ou la mesure automatique d'un instrument (700) selon l'une des trois revendications précédentes comportant un système de navigation (600) qui est relié sans fil ou par fil à l'unité de traitement de données (400) du dispositif d'analyse, dans lequel les informations déterminées relatives à l'instrument, en particulier la géométrie de l'élément fonctionnel (900) et du système de référence (800) ainsi que leur position relative, sont envoyées au système de navigation (600), dans lequel on peut procéder à une navigation avec le système de navigation (600), à l'aide des informations transmises relatives à l'agencement des repères formant le système de référence, en incluant et éventuellement en compensant les erreurs déterminées des repères.

18. Dispositif pour la vérification, l'étalonnage ou la mesure automatique d'un instrument (700) qui est utilisé pour la mise en oeuvre du procédé selon l'une des revendications 1 à 11 avec des instruments qui sont équipés d'une mémoire de données mobile, uniquement de lecture ou de lecture et d'enregistrement, utilisant en particulier des technologies de transmission sans fil, telles que RFID, pour fournir les caractéristiques d'identification nécessaires aux procédés suivant l'invention, et pour pouvoir éventuellement mémoriser sur l'instrument les résultats après mise en oeuvre du procédé suivant l'invention.

19. Programme d'ordinateur selon la revendication 12 qui détermine automatiquement les structures géométriques caractéristiques de l'instrument (700), en particulier la forme des éléments fonctionnels (900) et du système de référence (800) ou les repères formant le système de référence, à l'aide d'un algorithme de reconnaissance suivant l'invention, ce qui permet une identification et permet d'appeler de la banque de données les informations nécessaires à la mise en oeuvre de la vérification, de l'étalonnage ou de la mesure automatique de l'instrument, la reconnaissance automatique des structures géométriques caractéristiques pouvant également être utilisée pour soumettre à l'utilisateur, à l'aide du dispositif d'affichage, des propositions pour les zones à analyser avec une grande fidélité de détail.
